# EUROPÄISCHE PATENTSCHRIFT

(11) **EP 0 480 344 B1**
(45) Veröffentlichungstag und Bekanntmachung des Hinweises auf die Patenterteilung: **19.07.1995**
(21) Anmeldenummer: 91117016.5
(22) Anmeldetag: 07.10.1991
(51) Int. Cl.: A61F 15/00

(54) **Von Hand betreibbare Wickelvorrichtung**
Hand-operated winding device
Dispositif d'enroulement actionné à la main

(30) Priorität: 10.10.1990 DE 9014061 U
(43) Veröffentlichungstag der Anmeldung: 15.04.1992
(73) Patentinhaber: EVINA VERTRIEBS-GmbH & Co. KG, D-32051 Herford (DE)
(72) Erfinder: Schmitt, Margot, W-4900 Herford (DE)
(74) Vertreter: Stracke, Alexander, Dipl.-Ing.

(56) Entgegenhaltungen:
- DE-A- 3 000 513
- DE-U- 8 711 540

## Beschreibung

Die vorliegende Erfindung betrifft eine von Hand betreibbare Wickelvorrichtung für bandförmiges Wickelgut, beispielsweise Binden für medizinische Zwecke, mit einem rohrförmigen, einseitig mittels einer kreisrunden Stirnwand verschlossenen Außenmantel, der eine in der Umfangswand vorgesehne Öffnung aufweist, und mit einem drehbaren Innenmantel, der im wesentlichen aus einer Halbschale und einer der Stirnwand des Außenmantels gegenüberliegenden Bordscheibe besteht, wobei zur Begrenzung der beiden Endstellungen des Innenmantels innenseitig an der Stirnwand des Außenmantels zwei Anschläge angesetzt sind.

Bei einer aus der DE 30 00 513 A1 bekannten Wickelvorrichtung sind die die Endstellungen des Innenmantels begrenzenden Anschläge so gesetzt, daß in der schließstellung die Öffnung im Außenmantel vollständig geschlossen ist. Beim Aufwickeln des Wickelgutes wird der Innenmantel so verdreht, daß die Öffnung völlig frei ist. Dadurch wird das Wickelgut nicht in einer breitgestreckten Form der Wickelvorrichtung zugeführt. Dies trifft insbesondere bei medizinischen Binden zu, die nach dem Waschen zusammengeknautscht sind. Bei der vorbekannten Wickelvorrichtung wird der Innenmantel bedarfsweise so gedreht, daß die Öffnung der Umfangswand soweit verschlossen wird, daß ein Schlitz von verhältnismäßig geringer Breite entsteht, durch den das Wickelgut läuft. Naturgemäß ist es nun so, daß während des Wickelns der Innenmantel ungewollt verdreht wird, so daß das Wickelgut nicht gestreckt wird und es zu Überlappungen und zu einer Faltenbildung kommt.

Der vorliegenden Erfindung liegt die Aufgabe zugrunde, eine Wickelvorrichtung der gattungsgemäßen Art so weiterzuentwickeln, daß das Wickelgut faltenfrei und ohne Überlappungen unter Einhaltung einer bestimmten Breite des Schlitzes aufgewickelt werden kann.

Die gestellte Aufgabe wird erfindungsgemäß dadurch gelöst, daß in der durch einen Anschlag vorgegebenen Schließstellung des Innenmantels zwischen den einander zugewandten Kanten des Innenmantels und der Öffnung des Außenmantels ein Schlitz von stets gleichbleibender Breite gebildet ist.

Der Innenmantel läßt sich nunmehr in der Schließstellung nur soweit verdrehen, daß zwischen den einander zugewandten Kanten des Innenmantels und der Öffnung der Schlitz eine stets gleichbleibende Breite hat. Die Kanten verlaufen parallel zur Mittellängsachse der Wickelvorrichtung. Der gebildete Schlitz ist im Sinne einer Breitstreckeinrichtung für das Wickelgut zu sehen. Da die Wickelvorrichtung mit einer Hand gehalten und mit der anderen Hand die Wickelachse gedreht wird, ist es möglich, daß man mit einem Finger oder mit dem Daumen der die Wickelvorrichtung haltenden Hand den Innenmantel gegen den Anschlag drückt. Beim Aufwickeln wird das Wickelgut straff gezogen. Da das Wickelgut über die Kante des Innenmantels läuft, erfolgt das Aufwickeln mit einer Vorspannung des Wickelgutes, da die Reibungskräfte zu überwinden sind. Dadurch wird eine feste Rolle gewickelt. Durch die erfindungsgemäße Lösung wird nicht nur die Handhabung der Wickelvorrichtung verbessert, sondern auch das Produkt.

Da der Drehwinkel des Innenmantels nur so groß sein muß, daß zum Einfädeln des Wickelgutanfanges die Öffnung vollkommen freigegeben wird, ist in weiterer Ausgestaltung vorgesehen, daß in der zweiten Endstellung des Innenmantels die Öffnung des Außenmantels vollständig frei ist. Da die einzelnen Teile der Wickelvorrichtung als Kunststoffspritzgrußteile ausgebildet sind, sind die Anschläge mit dem Außenmantel ein einstückiges Teil. Die Anschläge liegen auf einem dem Durchmesser der Halbschale des Innenmantels entsprechenden Kreisbogen. Zweckmäßigerweise beträgt der Drehwinkel des Innenmantels im wesentlichen 30 Grad.

Anhand der beiliegenden Zeichnungen wird die Erfindung noch näher erläutert. Es zeigen:
- Fig. 1: eine erfindungsgemäße Wickelvorrichtung in Explosivdarstellung und
- Fig. 2 und 3: einen Vertikalschnitt des Außenmantels längs der Linie II/III-II/III, wobei die beiden Endstellungen des Innenmantels in strichpunktierten Linien angedeutet sind.

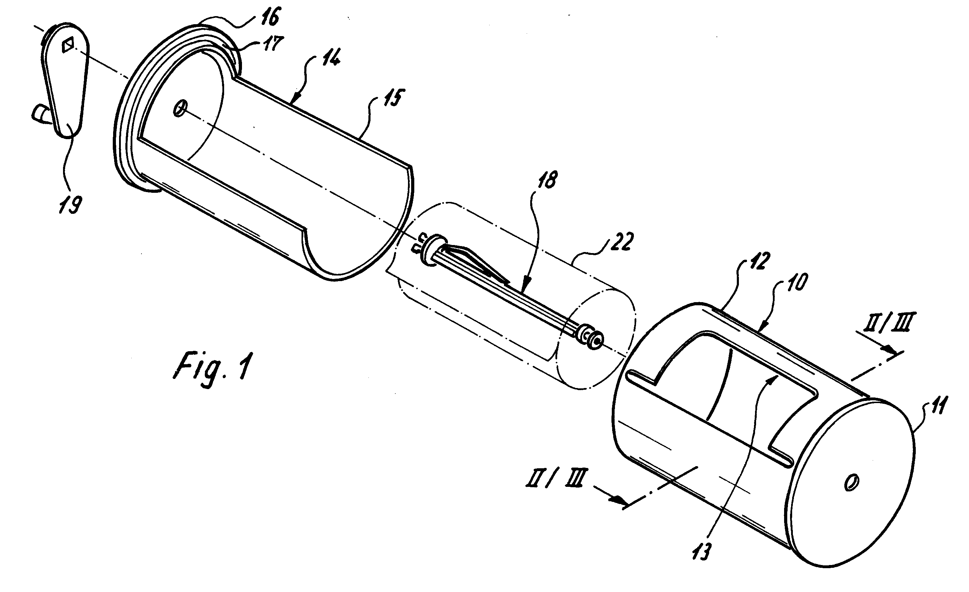

Die in der Fig. 1 aufgezeigte Vorrichtung besteht aus einem Außenmantel 10 mit kreisrundem Querschnitt. Die Umfangswand 12 des Außenmantels 10 weist eine Öffnung 13 auf, die sich annähernd über die gesamte Länge der Umfangswand 12 erstreckt. Außerdem erstreckt sich die Öffnung 13 über einen relativ großen Winkelbereich, wobei jedoch dieser Winkel ein spitzer Winkel ist, der im dargestellten Ausführungsbeispiel ca. 30° beträgt. Die Vorrichtung weist außerdem einen Innenmantel 14 auf, der aus einer Halbschale 15 und aus einer Bordscheibe 16 besteht. Der Außendurchmesser der Halbschale 15 entspricht dem Innendurchmesser des Außenmantels 10, so daß der Innenmantel 14 schließend in den Außenmantel 10 eingesteckt werden kann. Innenseitig ist die Bordscheibe 16 mit einer sich im wesentlichen über den Winkelbereich der Öffnung 13 erstreckenden Nut 17 versehen, in die der der Stirnwand 11 gegenüberliegende Rand des Außenmantels 10 geführt ist.

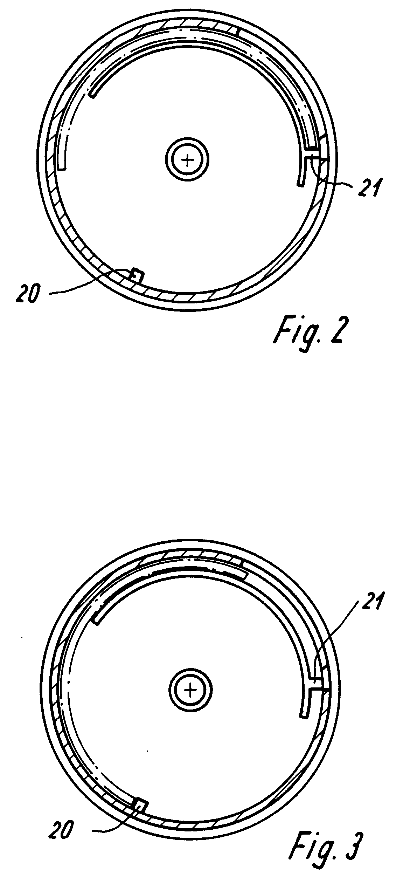

Die Wickelvorrichtung ist außerdem noch mit einer nicht näher erläuterten Wickelwelle 18 ausgerüstet, die in zentrischen Bohrungen der Stirnwand 11 des Außenmantels 10 und der Bordscheibe 16 des Innenmantels 14 gelagert ist. Ein Ende der Wickelwelle 18 steht gegenüber der Außenfläche der Bordscheibe 16 so weit vor, daß zum Drehen der Wickelwelle 18 auf dieses Ende eine Kurbel 19 aufgesteckt werden kann. Wie aus den Fig. 2 und 3 deutlich erkennbar, sind an der Innenseite der Stirnwand 11 des Außenmantels 10 zwei in einem Winkelabstand zueinander stehende Anschläge 20,21 angeformt. Der Anschlag 21 ragt um seine Breite in die Öffnung 13 hinein. Der andere Anschlag 20 steht in einem solchen Winkelversatz, daß die Öffnung 13 des Außenmantels 10 vollkommen freigegeben wird, wenn die zugeordnete Kante dagegenschlägt. Diese Darstellung ist der Fig. 3 gezeigt. Die Fig. 2 zeigt, daß ein Schlitz von der Breite des Anschlages 21 entsteht, wenn die Kante der Halbschale 15 dagegenschlägt. Die Fig. 2 ist die Gebrauchs- bzw. Wickelstellung. Aus dieser Figur ergibt sich, daß durch leichten Druck mit einem Finger oder einem Daumen die Halbschale 15 gegen den Anschlag 21 gedrückt werden kann. Der Außenmantel 10, der Innenmantel 14, die Wickelwelle 18 und die Kurbel 19 sind einstückige Kunststoffteile. Zum Herausnehmen des in strichpunktierten Linien dargestellten Wickels 22 wird der Innenmantel 14 aus dem Außenmantel 10 herausgezogen. Der Wickel 22 kann dann von der Wickelwelle 18 abgezogen werden.

## Patentansprüche

1. Von Hand betreibbare Wickelvorrichtung für bandförmiges Wickelgut, beispielsweise Binden für medizinische Zwecke, mit einem rohrförmigen, einseitig mittels einer kreisrunden Stirnwand (11) verschlossenen Außenmantel (10), der eine in der Umfangswand vorgesehene Öffnung (13) aufweist und mit einem drehbaren Innenmantel (14), der im wesentlichen aus einer Halbschale (15) und einer der Stirnwand (11) des Außenmantels (10) gegenüberliegenden Bordscheibe (16) besteht, wobei zur Begrenzung der beiden Endstellungen des Innenmantels (14) innenseitig an der Stirnwand (11) des Außenmantels (10) zwei Anschläge (20, 21) angesetzt sind, **dadurch gekennzeichnet, daß** in der durch einen Anschlag (21) vorgegebenen Schließstellung des Innenmantels (14) zwischen den einander zugewandten Kanten des Innenmantels (14) und der Öffnung (13) des Außenmantels (10) ein Schlitz von stets gleichbleibender Breite gebildet ist.

2. Von Hand betreibbare Wickelvorrichtung nach Anspruch 1, **dadurch gekennzeichnet, daß** in der zweiten Endstellung des Innenmantels (14) die Öffnung (13) des Außenmantels (10) vollständig frei ist.

3. Von Hand betreibbare Wickelvorrichtung nach Anspruch 1 oder 2, **dadurch gekennzeichnet, daß** der Außenmantel (10) und der Anschlag (21) bzw. die Anschläge (20, 21) als ein einstückiges Kunststoffteil ausgebildet sind.

4. Von Hand betreibbare Wickelvorrichtung nach einem oder mehreren der vorhergehenden Ansprüche 1 bis 3, **dadurch gekennzeichnet, daß** der Drehwinkel des Innenmantels (14) im wesentlichen 30 Grad beträgt.

## Claims

1. A manually operable winding apparatus for material to be wound, which is in strip form, for example bandages for medical purposes, comprising a tubular outer casing (10) which is closed at one end by means of a round end wall (11) and which has an opening (13) provided in the peripheral wall, and a rotatable inner casing (14) which essentially comprises a half-shell portion (15) and a flanged disc (16) which is disposed opposite the end wall (11) of the outer casing (10), wherein two stops (20, 21) are fitted on the inside to the end wall (11) of the outer casing (10) to define the two limit positions of the inner casing (14), characterised in that a slot of a width which is always uniform is formed between the mutually facing edges of the inner casing (14) and the opening (13) of the outer casing (10) in the closed position of the inner casing (14), which is predetermined by a stop (21).

2. A manually operable winding apparatus according to claim 1 characterised in that the opening (13) in the outer casing (10) is completely free in the second limit position of the inner casing (14).

3. A manually operable winding apparatus according to claim 1 or claim 2 characterised in that the outer casing (10) and the stop (21) or the stops (20, 21) are in the form of an integral plastics component.

4. A manually operable winding apparatus according to one or more of preceding claims 1 to 3 characterised in that the angle of rotary movement of the inner casing (14) is substantially 30°.

## Revendications

1. Dispositif d'enroulement, actionnable à la main, pour un produit à enrouler en forme de bande, par exemple pour des bandes à usage médical, comportant une enveloppe extérieure (10) tubulaire, fermée d'un côté par une paroi frontale (11) de forme circulaire, enveloppe qui présente une ouverture (13) prévue dans la paroi circonférentielle, et comportant une enveloppe intérieure (14) rotative constituée essentiellement d'une demi-coque (15) et d'une poulie à rebord (16) opposée à la paroi frontale (11) de l'enveloppe extérieure (10), deux butées (20, 21) étant disposées, sur la face intérieure, sur la paroi frontale (11) de l'enveloppe extérieure (10), afin de limiter les deux positions terminales de l'enveloppe intérieure (14), caractérisé en ce qu'en position de fermeture de l'enveloppe intérieure (14), donnée par une butée (21), une fente de largeur constante est formée entre les arêtes, orientées l'une vers l'autre, de l'enveloppe intérieure (14) et l'ouverture (13) de l'enveloppe extérieure (10).

2. Dispositif d'enroulement actionnable à la main selon la revendication 1, caractérisé en ce que, dans la deuxième position terminale de l'enveloppe intérieure (14), l'ouverture (13) de l'enveloppe extérieure (10) est complètement dégagée.

3. Dispositif d'enroulement actionnable à la main selon la revendication 1 ou 2, caractérisé en ce que l'enveloppe extérieure (10) et la butée (21) ou les butées (20, 21) sont constituées en une pièce en matière synthétique monobloc.

4. Dispositif d'enroulement actionnable à la main selon l'une ou plusieurs des revendications 1 à 3, caractérisé en ce que l'angle de rotation de l'enveloppe intérieure (14) représente sensiblement 30 degrés.
